# EUROPÄISCHE PATENTSCHRIFT

(11) **EP 3 302 599 B1**
(45) Veröffentlichungstag und Bekanntmachung des Hinweises auf die Patenterteilung: **22.09.2021**
(21) Anmeldenummer: 16726764.0
(22) Anmeldetag: 18.04.2016
(51) Int. Cl.: A61M 1/00, A61M 27/00

(54) **EINRICHTUNG ZUR DRAINAGE, INSBESONDERE ZUR DRAINAGE EINES CHRONISCHEN SUBDURALEN HÄMATOMS**
DEVICE FOR DRAINAGE, IN PARTICULAR FOR DRAINAGE OF A CHRONIC SUBDURAL HEMATOMA
DISPOSITIF POUR LE DRAINAGE, EN PARTICULIER POUR LE DRAINAGE D'UN HÉMATOME SOUS-DURAL CHRONIQUE

(30) Priorität: 02.06.2015 DE 102015108754
(43) Veröffentlichungstag der Anmeldung: 11.04.2018
(73) Patentinhaber: Primed Halberstadt Medizintechnik GmbH, 38820 Halberstadt (DE)
(72) Erfinder: SPIEGELBERG, Andreas, 8810 Horgen (CH)
(74) Vertreter: Donath, Dirk
(86) Internationale Anmeldenummer: PCT/DE2016/100183
(87) Internationale Veröffentlichungsnummer: WO 2016/192705

(56) Entgegenhaltungen:
- EP-A2- 2 777 751
- WO-A2-2007/005851
- DE-T5-112012 005 513
- US-A- 5 665 070

## Beschreibung

Die Erfindung betrifft eine Einrichtung zur Drainage, insbesondere zur Drainage eines chronischen subduralen Hämatoms (CSH).

Das Chronische Subdurale Hämatom ist einer Erkrankung, die typischerweise bei älteren Patienten durch ein traumatisches Ereignis ausgelöst wird. Es kommt zu einer Blutung unter der harten Hirnhaut (Dura) in den subduralen Raum (den Raum darunter). Die Blutung kommt meist unter dem Druck in dem Bluterguss (Hämatom) zum Stehen, es blutet aber in dem Maße, wie das Blut abgebaut wird, nach. Es handelt sich also um eine chronische Erkrankung, die nur chirurgisch beherrscht werden kann. Dazu wird der Schädel geöffnet, das Blut aus dem Bluterguss wird abgelassen oder je nach Größe auch ausgeräumt oder ausgewaschen. Nach diesem Eingriff besteht eine hohe Wahrscheinlichkeit des Nachblutens und des Wiederauftretens des Zustandes. Durch klinische Studien konnte belegt werden, dass die Wahrscheinlichkeit des Wiederauftretens signifikant kleiner ist, wenn nach dem chirurgischen Eingriff ein Drainagekatheter eingelegt wird, der über einige Tage weiteres Blut abdrainiert. Eine solche Drainage wird typischerweise schwerkraftinduziert angelegt, indem ein Auffangbehälter (Reservoir, Tropfkammer, Beutel) einige Zentimeter unter dem Niveau des Hämatoms angebracht wird, so dass unter hydrostatisch erzeugtem geringem Unterdruck das Blut und eventuelle Wundflüssigkeit abgeführt werden.

Die Schwierigkeit dabei besteht darin, dass die Wundhöhle nicht vollständig mit Flüssigkeit gefüllt ist, so dass auch Luft angesaugt werden kann. Diese bildet dann im Schlauchsystem Blasen oder Schaum und die Schwerkraftdrainage kommt zum Erliegen.

Es ist versucht worden, echte Saugdrainagen zu verwenden, bei denen entweder mechanisch durch federbelastete Balgen o. ä. oder durch Vakuumflaschen gesaugt wird. Unterdrücke in einer Größenordnung von mehr als -30 mmHg (-4 kPa) sind aber für das Hirn schädlich und nicht zulässig.

Vakuumbehälter mit derart niedrigem Unterdruck saugen aber jeweils nur einen geringen Teil (weniger als 5%) Ihres Volumens, bevor das Vakuum erschöpft ist. Balgenkonstruktionen erzeugen konstruktionsbedingt einen über das Arbeitsvolumen bis auf Null abfallenden Unterdruck, so dass ebenfalls nicht das gesamte Volumen für die Drainage zur Verfügung steht.

Weiterhin sind Produkte bekannt, bei denen zwei Platten, die mit einer schlauchförmigen Membran verbunden sind, durch eine oder mehrere Schraubenfedern auseinander gedrückt werden, so dass im Inneren ein Unterdruck entsteht. Auch solche Produkte erzeugen einen Unterdruck, der von seinem Maximalwert auf Null abfällt, wenn das Produkt so ausgelegt ist, dass die Federn in der Endlage vollständig entspannt sind. Es ist versucht worden, durch Vorspannung der Federn den Unterdruck in der Endlage nicht auf Null, sondern auf einen durch die Vorspannung definierten Wert, der ungleich Null ist, abfallen zu lassen. Die erreichbare Vorspannung ist aber aus konstruktiven Gründen begrenzt und die Änderung des Unterdruckes über den Arbeitsbereich ist immer noch zu groß.

Ein weiteres Problem entsteht, wenn nach dem chirurgischen Eingriff ein Liquorleck auftritt. Das ist eine Situation, in der Hirnwasser (Liquor cerebrospinalis, kurz: Liquor) austritt. Bei unkontrollierter Saugdrainage, selbst mit niedrigem Druck, kann Liquor in größerer Menge angesaugt werden, was zu Schäden für den Patienten bis zum Tod führen kann.

Die Schrift WO 2007/0058851 A2 offenbart die Verwendung von Vorspannelementen, um einen kollabierten, expandierbaren Behälter zu öffnen.

Dabei wird gelehrt, dass eine Vorspannung durch ein Vorspannelement, wie beispielsweise eine mechanische Vorspannung (Feder, elastisch, etc.), eine pneumatische Vorspannung (Aufbringen von Luftdruck), eine elektrische Vorspannung (Elektromagnet oder motorisiertes Vorspannelement) oder dergleichen oder eine beliebige Kombination davon erzeugt wird, um den ausdehnbaren Behälter zu öffnen.

Die Besonderheit dieser technischen Lösung besteht darin, dass die Vorspannung dem Behälter im Zustand des minimierten Volumens aufgeprägt wird, denn durch sie soll der Behälter expandieren.

EP 2 777 751 A2 offenbart ein Werkzeug umfassend einen Lokalisierer mit einer Mitte, die mit einer Rotorachse ausgerichtet ist, wenn sie über dem Ventil angeordnet ist. Der Lokalisierer ist ungefähr senkrecht zur Rotorachse. Ein Einstellelement ist in dem Lokalisierer angeordnet. Die Einstellvorrichtung passt in die Wand und hat einen Magneten mit einer magnetischen Achse, um den Rotor zu entriegeln. Eine einstellbare äußere Wand hat eine erste Positionsabmessung, die es dem Einstellelement erlaubt, sich um das Zentrum des Lokalisierers zu drehen und die magnetische Achse mit der Mitte auszurichten. Die einstellbare äußere Wand hat eine zweite Positionsabmessung weniger als die erste Dimension und die erste Positionsabmessung. Der Justierer kann sich seitlich, rotatorisch und orbital in dem Lokalisierer bewegen, der die magnetische Achse mit der Mitte falsch ausrichtet. Durch falsche Ausrichtung der Magnet- und Rotorachsen werden die Rotoren entriegelt und der Magnet behält den entriegelten Zustand bei, auch wenn die Achsen falsch ausgerichtet sind.

DE 11 2012 005513 T5 offenbart eine medizinische Absaugvorrichtung und insbesondere eine medizinische Absaugvorrichtung mit einer Bodenplatte, einer Druckplatte, die in einem gewissen Abstand von der Bodenplatte angeordnet ist und ein Ablassloch definiert, das durch eine Öffnungs- / Schließeinrichtung geöffnet und geschlossen wird, eine Schraubenfeder, die zwischen der Bodenplatte und der Druckplatte installiert ist, eine Dichtungsmembran, die die Kanten der Bodenplatte und der Druckplatte verbindet und einen Aufnahmeraum definiert, der Körperflüssigkeit zwischen der Bodenplatte und der Druckplatte aufnehmen kann, und ein Drainagerohr, das mit der Druckplatte verbunden ist, um von dem Körper erzeugtes Körperfluid zu dem Aufnahmeraum zu leiten, und ein Rückschlagventil enthält, das es dem Fluid ermöglicht, nur in der Richtung von dem Körper zu dem Aufnahmeraum zu fließen, wobei der medizinische Aspirator die Wirkung hat, eine Kontamination der Körperflüssigkeit durch die Außenluft zu verhindern, die aus dem Körper extrahiert und in dem Aufnahmeraum gespeichert wird.

Nachteilig bei diesen technischen Lösungen eines Druckausgleichsventils ist, dass dieses Ventil einen Druckunterschied nur ausgleichen, aber nicht aufbauen kann.

Aufgabe der Erfindung ist die Schaffung einer Einrichtung zur Drainage des chronischen subduralen Hämatoms unter Verwendung eines Reservoirs, das konstruktionsbedingt einen annähernd konstanten Unterdruck insbesondere in einer Größenordnung von unter 30 mmHg (4 kPa) erzeugt.

Eine weitere Aufgabe der Erfindung besteht insbesondere darin, eine Einrichtung der genannten Art zu schaffen, bei der der Volumenstrom, also das pro Zeit abgesaugte Volumen, begrenzt ist.

Dieses Problem wird durch eine Einrichtung mit den Merkmalen des Anspruchs 1 gelöst. Vorteilhafte Ausgestaltungen der Erfindung sind in den Unteransprüchen angegeben und werden nachfolgend beschrieben. Die nachfolgend beschriebenen Merkmale der Erfindung können in jeglicher sinnvoller Art und Weise miteinander kombiniert werden.

Erfindungsgemäß wird das Problem durch die Kombination eines schlauchförmigen Reservoirs mit biegeweicher Wand und steifem Deckel und Boden mit wenigstens einer innen oder außen liegenden Feder gelöst. Die wenigstens eine Feder wird so angeordnet, dass sie Boden und Deckel auseinander drückt bzw. voneinander weg bewegen kann. Die wenigstens eine Feder ist hierbei vorzugsweise derart vorgespannt, dass sie nur über einen Teil ihres Federweges verformt wird, so dass ihre Kraft, und damit der von ihr erzeugte Unterdruck sich nur wenig ändert. Weiterhin ist erfindungsgemäß wenigstens eine Anordnung von einem oder mehreren Magneten vorhanden, die die Kennlinie der wenigstens einen Feder so kompensiert, dass die besagte Kraft annähernd konstant gehalten wird.

Mit anderen Worten ist also die mindestens eine Feder gemäß einer Ausführungsform der Erfindung vorzugsweise so vorgespannt, dass der Weg zwischen der Anfangslage und einer durch die Länge des Reservoirs vorgegebenen Endlage der mindestens einen Feder nur einen Teil des gesamten Federweges der mindestens einen Feder ausmacht. Die besagte Länge des Reservoirs ist hierbei dessen maximale Ausdehnung in der Bewegungsrichtung der Feder. Weiterhin sind erfindungsgemäß ein oder mehrere Magnete vorgesehen, die so konfiguriert sind, dass sie - neben der Federkraft - eine zusätzliche Kraft auf das Reservoir ausüben, derart, dass eine resultierende, den Deckel und den Boden auseinanderdrückende Kraft über jenen Teil des Federweges im Wesentlichen konstant ist bzw. bevorzugt konstant ist. Hierbei ist eine Vielzahl an geeigneten Magnetanordnungen möglich, wobei besonders bevorzugte Anordnungen weiter unten angegeben sind.

Im Wesentlichen konstant heißt hierbei, dass der Betrag der Kraft über jenen Teil des Federweges bevorzugt nicht mehr als 50%, bevorzugt nicht mehr als 40%, bevorzugt nicht mehr als 30%, bevorzugt nicht mehr als 20%, bevorzugt nicht mehr als 10%, bevorzugt nicht mehr als 5%, bevorzugt nicht mehr als 1% von dem Durchschnittsbetrag der Kraft über jenen Teil des Federweges abweicht.

In einer alternativen Ausführungsform heißt "im Wesentlichen konstant", dass der Kraft ein Unterdruck im Reservoir entspricht, der über jenen Teil des Federweges der mindestens einen Feder zwischen Anfangs- und Endlage der mindestens einen Feder im Bereich von 20 bis 40 mmHg (2,67 bis 5,33 kPa) liegt, vorzugsweise in einem Bereich von 25 bis 35mmHg (3,33 bis 4,66 kPa), vorzugsweise in einem Bereich von 27 bis 33mmHg (3,60 bis 4,40 kPa), vorzugsweise in einem Bereich von 28 bis 32mmHg (3,73 bis 4,27 kPa), vorzugsweise in einem Bereich von 29 bis 31mmHg (3,87 bis 4,13 kPa). In einer weiteren Ausgestaltung der Erfindung gelten diese bevorzugten Druckbereiche ebenfalls, wobei hier immer die oberen Grenze durch 30mmHg (4 kPa) gebildet wird.

In einer bevorzugten Ausführung der Erfindung ist das schlauchförmige Reservoir mit biegeweicher Wand und steifem Deckel und Boden mit wenigstens einer innenliegenden Feder in einem Gehäuse angeordnet. Vorzugsweise ist das schlauchförmige Reservoir mit einem oder mehreren Anschlüssen für je einen Schlauch versehen, wobei über jene Anschlüsse je ein Schlauch in Strömungsverbindung mit dem Reservoir bringbar ist.

Jene Anschlüsse durchdringen bevorzugt eine Wand des Gehäuses und sind mit dieser und dem Reservoir dicht verbunden oder in diese mittels Dichtmitteln eingedichtet.

Weiterhin ist gemäß einer Ausführungsform der Erfindung das Gehäuse mit wenigstens einem Anschluss versehen. Wenn der Anschluss in dem Gehäuse geöffnet ist, kann sich das schlauchförmige Reservoir frei ausdehnen und zusammenziehen. Ist der Anschluss geschlossen, wird das Ausdehnen und Zusammenziehen blockiert, da sich in einem Innenraum des Gehäuses, insbesondere zwischen einer Wand des Gehäuses und einer Wandung bzw. dem Deckel des schlauchförmigen Reservoirs ein Über- oder Unterdruck aufbaut, der die Bewegung der Wand des schlauchförmigen Reservoirs behindert.

In einer bevorzugten Ausführung der Erfindung ist in dem Anschluss in dem Gehäuse eine Durchflussbegrenzung vorgesehen, z.B. in Form eines Strömungswiderstandes, z.B. einer semipermeablen Membran, einer Düse oder einer Lochplatte, welche den Abstrom von Umgebungsluft aus dem Raum zwischen der Gehäusewand und jener Wandung des schlauchförmigen Reservoirs begrenzt.

In einer besonders bevorzugten Ausführung der Erfindung ist der Boden des schlauchförmigen Reservoirs mit einer (insbesondere unteren) Wand des Gehäuses form- kraft- oder stoffschlüssig so verbunden, dass bei Zusammenziehen oder Ausdehnen des schlauchförmigen Reservoirs der dem Boden in Bewegungsrichtung der mindestens einen Feder gegenüberliegende Deckel des Reservoirs eine relative Bewegung zum Gehäuse ausführt.

Weiterhin ist gemäß einer Ausführungsform der Erfindung im oder am Deckel wenigstens ein Magnet angebracht. Weiterhin ist in der Wand des Gehäuses, die dem Deckel gegenüberliegt, wenigstens ein Magnet oder ein paramagnetischer, bevorzugt superparamagnetischer oder ferromagnetischer, Körper angebracht, welcher so orientiert ist, dass er bei Annäherung des Deckels an die gegenüberliegende Wand eine grösser werdende Anziehungskraft ausübt. Bei abnehmender Kraft der wenigstens einen Feder, wird also eine zunehmende Magnetkraft ausgeübt, welche die Abnahme der Federkraft ganz oder teilweise kompensiert. Weiterhin kann die Anordnung auch andersherum ausgeführt werden, d.h. ein etwaiger paramagnetischer, superparamagnetischer oder ferromagnetischer Körper auch am Deckel angeordnet sein und der mindestens Magnet an jener Wand des Gehäuses.

Vorzugsweise wirken der oder die Magnete bzw. paramagnetischer, superparamagnetischer oder ferromagnetischer Körper so zusammen, dass eine resultierende, den Boden und den Deckel auseinander bewegende Kraft im Wesentlichen konstant ist, bevorzugt konstant ist (siehe auch oben).

Zum Spannen der Einrichtung kann das Reservoir mittels einer Injektionsspritze o ä leergesaugt werden. In einer bevorzugten Ausführung der Erfindung wird die Einrichtung gespannt, indem der bewegliche Deckel von Hand gegen die Feder- und ggf. Magnetkraft bewegt wird.

Insbesondere im Falle der Anordnung des Reservoirs in einem vorzugsweise hermetisch dichten Gehäuse (z.B. mit oben beschriebenen Strömungswiderstand zur Volumenstrombegrenzung) wird gemäß einer Ausführungsform der Erfindung eine Öffnung in dem Gehäuse vorgesehen, durch die z.B. ein Stab oder Ähnliches gegen die bewegliche Platte gedrückt wird, wobei der Stab gegen das Gehäuse in einer zylindrischen Führung durch eine Dichtung abgedichtet ist.

In einer bevorzugten Ausführung der Erfindung ist eine Scheibe unter der Öffnung angebracht, die mit einem Balg oder einer elastischen Membran gegen das Gehäuse abgedichtet ist. Die Scheibe ist vorzugsweise vom Durchmesser her größer als die Öffnung, so dass sie in einer Ruheposition an einem umlaufenden Rand der Öffnung an der Innenwand des Gehäuses anliegen kann. Die Öffnung ist bevorzugt so dimensioniert, dass der Anwender einen Finger oder einen geeigneten Gegenstand durch die Öffnung stecken kann, wobei er oder sie die Scheibe von der Innenwand des Gehäuses weg gegen den beweglichen Deckel des Reservoirs drückt und damit die Einrichtung spannt.

In einer weiteren bevorzugten Ausführungsform der Erfindung ist zum Spannen eine elastische Pumpkammer in oder an einer Wand des Gehäuses vorgesehen, die über ein Rückschlagventil mit der Atmosphäre verbunden ist und über ein weiteres Rückschlagventil mit einem Innenraum des Gehäuses. Die Rückschlagventile sind so orientiert, dass sie eine Strömung von der Atmosphäre in die elastische Pumpkammer und aus der Pumpkammer in den Innenraum des Gehäuses erlauben und eine Strömung in Gegenrichtung sperren. Wird die Pumpkammer betätigt, wird Luft in den Innenraum des Gehäuses gepumpt und durch den sich aufbauenden Druck das Reservoir zusammengedrückt (gespannt).

Weiterhin ist gemäß einer Ausführungsform der Erfindung vorgesehen, dass die mindestens eine Feder eine Mehrzahl an Windungen aufweist und so an einer Peripherie des Deckels und des Bodens angeordnet ist, dass das Reservoir durch die Windungen der mindestens einen Feder gestützt wird.

Weiterhin ist gemäß einer Ausführungsform der Erfindung vorgesehen, dass die Einrichtung zumindest ein Spreizelement aufweist, um ein Kollabieren der biegeweichen Wand des Reservoirs zu verhindern, insbesondere wenn der Deckel in Richtung auf den Boden bewegt wird. Bei diesem Spreizelement kann es sich z.B.um einen mit der Wand des Reservoirs verbundenen Ring handeln.

Weiterhin ist gemäß einer Ausführungsform der Erfindung vorgesehen, dass das mindestens eine Spreizelement eine in einem Innenraum des Reservoirs angeordnete Platte ist, die sich vorzugsweise quer zur Bewegungsrichtung der mindestens einen Feder erstreckt.

Weitere Merkmale und Vorteile der Erfindung sollen nachfolgend bei der Beschreibung von Ausführungsbeispielen anhand der Figuren erläutert werden. Es zeigen:
- Fig. 1: eine geschnittene Ansicht einer erfindungsgemäßen Einrichtung,
- Fig. 2: eine geschnittene Ansicht der Einrichtung gemäß Figur 1 mit gespannter Feder,
- Fig. 3: eine geschnittene Ansicht einer weiteren erfindungsgemäßen Einrichtung,
- Fig. 4: eine geschnittene Ansicht einer weiteren erfindungsgemäßen Einrichtung mit mehreren Federn,
- Fig. 5 - 6: geschnittene Detailansichten einer Öffnung einer erfindungsgemäßen Einrichtung zum Spannen der Feder(n) der Einrichtung
- Fig. 7: einen graphische Darstellung des Verlaufs des Unterdruckes [mmHg] über dem angesaugten Volumen [ml] der Einrichtung nach Fig. 3 mit und ohne Magnete.

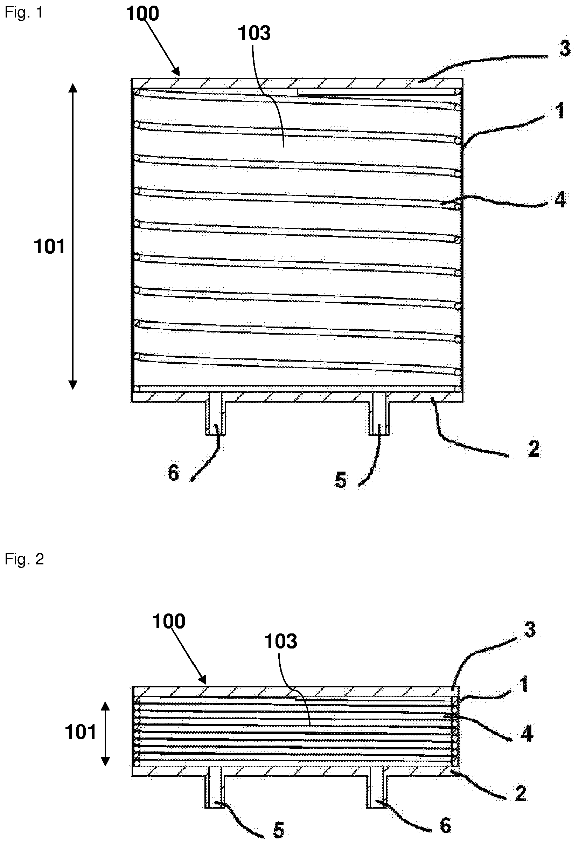

Die Figur 1 zeigt die prinzipielle Funktionsweise einer erfindungsgemäßen Einrichtung. In der dargestellten Ausführung weist die Einrichtung ein Reservoir 100 auf, das einen starren Boden 2 und einen dem Boden 2 gegenüberliegenden starren Deckel 3 aufweist. Der Boden 2 und der Deckel 3 sind über eine biegeweiche, umlaufende Wand 1 miteinander verbunden, so dass das besagte Reservoir 100 gebildet wird.

Vorliegend ist die biegeweiche Wand 1 des Reservoirs 100 z.B. als ein zylindrischer Schlauch aus dünner Kunststofffolie ausgeführt. Der Schlauch 1 ist dabei an seinen Enden durch den starren Boden 2 und einen starren Deckel 3 verschlossen.

Weiterhin ist in einem Innenraum 103 des Reservoirs 100 eine Feder 4, vorzugsweise in Form einer Schraubenfeder 4, angeordnet, die gegen den Boden und den Deckel vorgespannt ist, so dass sie versucht Boden 2 und Deckel 3 auseinander zu bewegen. Der Boden 2 und der Deckel 3 des Reservoirs 100 liegen also einander in der Bewegungsrichtung 101 der Feder 4 gegenüber.

Die Feder 4 ist dabei derart gegen den Boden 2 und den Deckel 3 vorgespannt, dass sie in der in Figur 1 gezeigten voll expandierten Position (Endlage) bereits auf vorzugsweise 50% bis 70%, vorzugsweise 60% ihrer entspannten Länge zusammengedrückt ist. In der in der Fig. 2 gezeigten, zusammengedrückten Position des Reservoirs 100 bzw. der Feder 4 ist die Feder 4 in ihrer Anfangslage vollständig zusammengedrückt. Ihr Federweg ist also insbesondere zu 50% bis 70% bzw. zu 60% ausgenützt.

Die Feder 4 ist bevorzugt weiterhin so ausgelegt, dass in der vollständig zusammengedrückten Position (vgl. Fig. 2) die dann maximal mögliche Federkraft den gewünschten Unterdruck erzeugt. In der in Fig. 1 gezeigten vollständig expandierten Position beträgt der Unterdruck 50% bis 70% bzw. 60% des gewünschten Wertes. Der Unterdruck ändert sich also nicht zwischen Null und 100%, sondern nur zwischen Null und 50% bis 70% bzw. Null und 60%. Bei dieser Konstruktion verhindern die Windungen der Feder 4 weiterhin ein Kollabieren des Reservoirs 100, insbesondere der biegeweichen Wand 1, beim Zusammendrücken des Reservoirs 100.

Weiterhin weist die Einrichtung bzw. das Reservoir 100 einen ersten Anschluss 5 auf, über den abdrainiertes Fluid in das Reservoir 100 einsaugbar ist, sowie einen zweiten Anschluss 6 über den besagtes Fluid aus dem Reservoir 100 abziehbar ist.

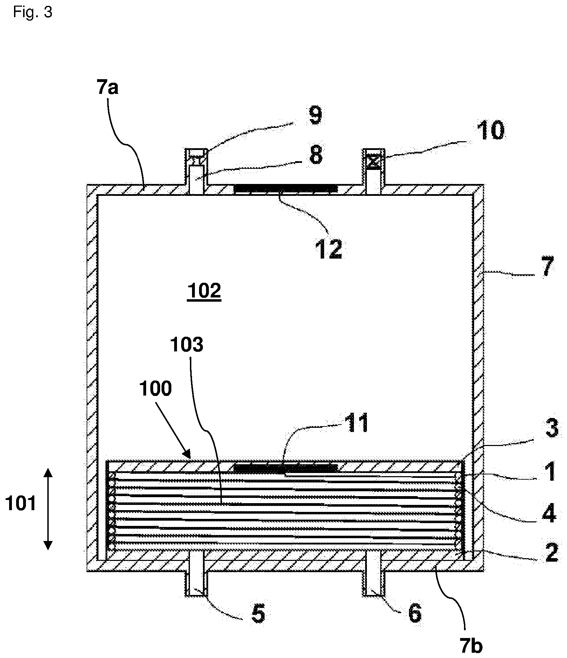

Fig. 3 zeigt eine weitere Ausführungsform einer erfindungsgemäßen Einrichtung, bei der das in den Figuren 1 und 2 gezeigte Reservoir 100 in einem Gehäuse 7 angeordnet ist.

Die beiden Anschlüsse 5 und 6 durchdringen bei dieser Ausfuhrungsform das Gehäuse 7.

Der Boden 2 ist mit einer Wand 7b des Gehäuses 7 verbunden, wohingegen der Deckel 3 beweglich in einem Innenraum 102 des Gehäuses 7 angeordnet ist.

Das Gehäuse 7 ist weiterhin mit einem Anschluss 8 versehen, in welchem ein Strömungswiderstand 9 angeordnet ist.

Weiterhin ist ein Ventil 10 vorhanden, welches, wenn geöffnet, das ungehinderte Einströmen von Luft in den Innenraum 102 des Gehäuses 7 erlaubt, und zwar zwischen Gehäuse 7 und einer Wandung 1, 3 des Reservoirs 100 erlaubt.

Weiterhin weist die Einrichtung gemäß Fig. 3 vorzugsweise einen Magneten 11 auf, der in dem beweglichen Deckel 3 des Reservoirs 100 angeordnet ist, sowie einen Magnet 12, welcher fest in der dem Deckel 3 gegenüberliegenden Wand 7a des Gehäuses 7 angeordnet ist.

Wenn sich die Feder 4 entspannt, ihre Kraft also abnimmt, nähert sich der Magnet 11 dem Magneten 12 an und übt eine zunehmende Kraft auf diesen aus, die die Abnahme der Federkraft kompensiert, so dass eine resultierende Kraft, die den Deckel 3 vom Boden 2 entfernt möglichst konstant ist (siehe oben).

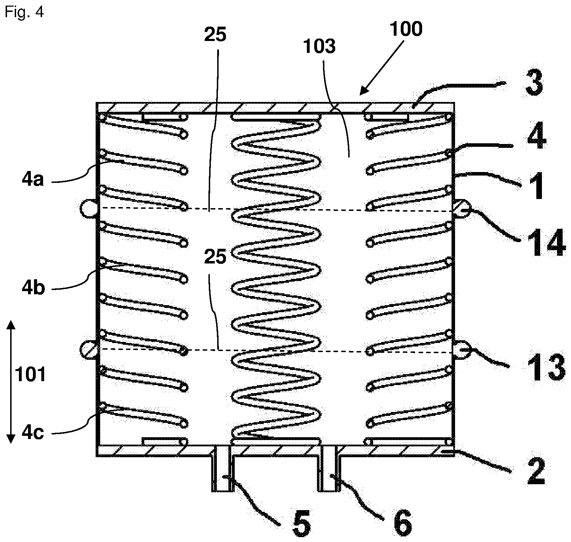

Die in der Fig. 4 gezeigte Ausführung der Einrichtung bzw. des Reservoirs 100 verwendet mehrere Federn, die zwischen den Rändern des Bodens 2 und des Deckels 3 angeordnet sind. Bei dieser Ausführung sind zusätzlich vorzugsweise zwei Spreizringe 13 und 14 an der umlaufenden Wand 1 angeordnet, die das Kollabieren des schlauchförmigen Reservoirs 100, z.B. ähnlich wie bei einem Balg oder einer Ziehharmonika, verhindern. Anstelle derartiger Spreizringe können auch eine oder mehrere Platten 25 im Innenraum des Reservoirs 100 vorgesehen sein, die sich quer zu den Federn 4 bzw. zur Bewegungsrichtung 101 erstrecken und an der Wand 1 anliegen. Derartige Platten 25 können von den Federn 4 durchdrungen werden. Die Federn 4 können jedoch auch jeweils entsprechend geteilt sein, wobei dann die resultierenden Federabschnitte 4a, 4b, 4c sich an den dann durchgehenden Platten 25 abstützen. Die z.B. anstelle der Spreizringe 13, 14 vorgesehenen Platten 25 sind in der Figur 4 mit gestrichelten Linien angedeutet.

Die in der Figur 4 gezeigte Konfiguration kann natürlich auch in einem Gehäuse 7 angeordnet werden, das z.B. wie oben beschrieben ausgeführt ist.

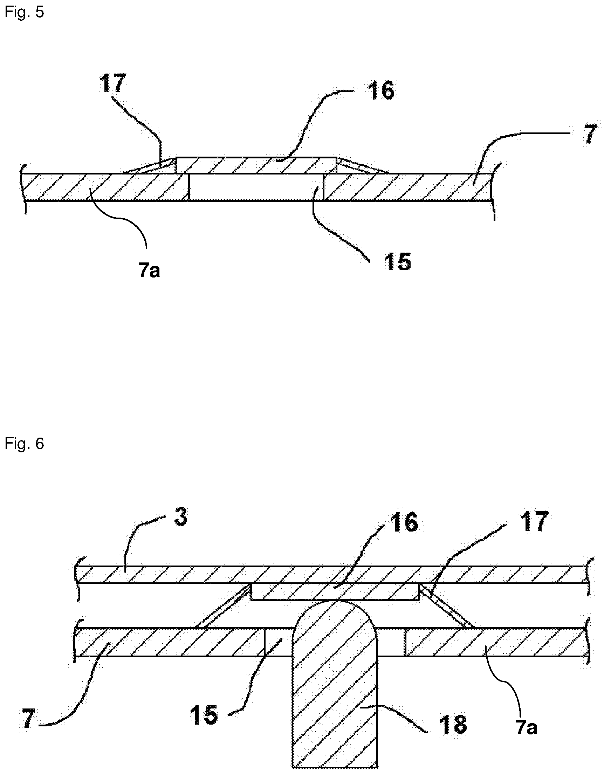

Weiterhin zeigt die Figur 5 im Detail eine mögliche Anordnung zum Spannen der Einrichtung, die bei allen Ausführungsformen mit Gehäuse 7 verwendet werden kann. In der dem Deckel 3 gegenüberliegenden Wand 7a des Gehäuses 7 ist dabei eine Öffnung oder Bohrung 15 angeordnet. Eine bewegliche Scheibe 16 ist im Innenraum 102 des Gehäuses 7 angeordnet und mit der Wand 7a des Gehäuses 7 durch eine elastische Membran 17 oder einen Balg 17 verbunden. Wenn, wie in Fig. 6 gezeigt, der Anwender oder die Anwenderin einen Finger 18 oder einen geeigneten Gegenstand in die Öffnung 15 steckt, kann er oder sie die Scheibe 16 gegen den Deckel 3 drücken und damit die Einrichtung bzw. deren Feder(n) 4 spannen.

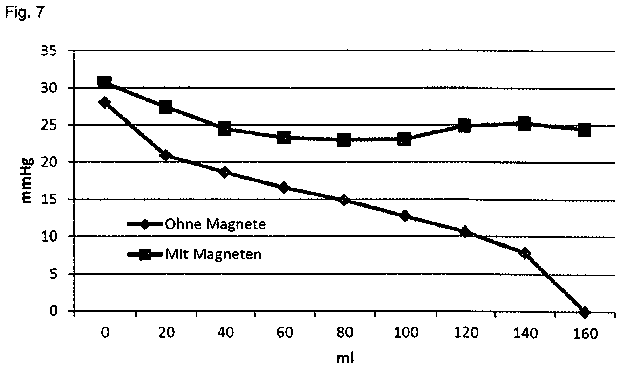

Schließlich zeigt Fig. 7 zeigt den Verlauf des Unterdruckes im Reservoir 100 in Abhängigkeit vom angesaugten Volumen der Einrichtung gemäß Fig. 3 mit und ohne Magnete 11, 12.

## Patentansprüche

1. Einrichtung zur Drainage, mit:
- einem schlauchförmigen Reservoir (100) aufweisend eine biegeweiche Wand (1) sowie einen steifen Deckel (3) und einen steifen Boden (2), und
- zumindest einer Feder (4), welche eine den Deckel (3) und den Boden (2) auseinanderdrückende Kraft ausübt, wobei der Deckel (3) in Richtung auf den Boden (2) bewegbar ist, um die Feder in eine Anfangslage zu bringen,
**dadurch gekennzeichnet,**
**dass** die Einrichtung einen oder mehrere Magnete (11, 12) aufweist, die so konfiguriert sind, dass sie eine zusätzliche Kraft auf das Reservoir (100) ausüben, derart, dass eine resultierende, den Deckel (3) und den Boden (2) auseinanderdrückende Kraft über jenen Teil des Federweges nicht mehr als 50%, 40%, 30%, 20%, 10%, 5%, oder 1% von dem Durchschnittsbetrag der Kraft über jenen Teil des Federweges abweicht oder der Kraft ein Unterdruck im Reservoir entspricht, der über jenen Teil des Federweges der Feder zwischen Anfangs- und Endlage der Feder im Bereich von 20 bis 40 mmHg (2,67 bis 5,33 kPa) liegt.

2. Einrichtung nach Anspruch 1, **dadurch gekennzeichnet, dass** die mindestens eine Feder (4) so vorgespannt ist, dass der Weg zwischen der Anfangslage und einer durch die Länge des Reservoirs (100) vorgegebenen Endlage der mindestens einen Feder (4) nur einen Teil des gesamten Federweges der mindestens einen Feder (4) ausmacht.

3. Einrichtung nach Anspruch 1 oder 2, **dadurch gekennzeichnet, dass** das schlauchförmige Reservoir (100) zumindest einen Anschluss (5, 6) zum Anschließen eines Schlauches aufweist.

4. Einrichtung nach Anspruch 3, **dadurch gekennzeichnet, dass** das Reservoir (100) in einem Gehäuse (7) der Einrichtung angeordnet ist, und dass der mindestens eine Anschluss (5, 6) das Gehäuse (7) durchdringt und dicht mit dem Gehäuse (7) und dem Reservoir (100) verbunden ist oder mittels Dichtmitteln in diese eingedichtet ist.

5. Einrichtung nach Anspruch 4, **dadurch gekennzeichnet, dass** das Gehäuse (7) zumindest einen Anschluss (8) aufweist, über den Umgebungsluft in einen Innenraum (102) des Gehäuses (7) zwischen einer Wand (7a) des Gehäuses (7) und einer Wandung (3) des Reservoirs (100) ein- und auslassbar ist.

6. Einrichtung nach Anspruch 5, **dadurch gekennzeichnet, dass** in dem mindestens einen Anschluss (8) ein Strömungswiderstand (9) angeordnet ist, welcher den Volumenstrom der Umgebungsluft begrenzt.

7. Einrichtung nach Anspruch 6, **dadurch gekennzeichnet, dass** der Strömungswiderstand (9) durch eines der folgenden Element gebildet ist:
- eine Düse,
- ein Element, das aus einer Mehrzahl von Düsen besteht,
- ein poröses Element,
- eine Membran, die eine oder eine Mehrzahl an Durchgangsöffnungen aufweist,
- eine Membran, die aus einem gasdurchlässigen Werkstoff besteht oder einen gasdurchlässigen Werkstoff aufweist,
- eine Membran, die aus Silikonkautschuk besteht oder Silikonkautschuk aufweist.

8. Einrichtung nach einem der vorhergehenden Ansprüche, **dadurch gekennzeichnet, dass** der Boden (2) des Reservoirs (100) fest mit einer Wand (7b) Gehäuses (7) verbunden ist und der Deckel (3) bezüglich des Gehäuses (7) bewegbar im Gehäuse (7) angeordnet ist.

9. Einrichtung nach Anspruch 8, **dadurch gekennzeichnet, dass** am Deckel (3) zumindest ein Magnet (11) angeordnet ist, und wobei an einer dem Deckel (3) gegenüberliegenden Wand (7a) des Gehäuses (7) zumindest ein weiterer Magnet (12) angeordnet ist, wobei der mindestens eine Magnet (11) und der mindestens eine weitere Magnet (12) so orientiert sind, dass sie sich gegenseitig anziehen.

10. Einrichtung nach Anspruch 8, **dadurch gekennzeichnet, dass** am Deckel (3) zumindest ein Magnet (11) angeordnet ist, und wobei an einer dem Deckel (3) gegenüberliegenden Wand (7a) des Gehäuses (7) zumindest ein Körper aus einem magnetischen, paramagnetischen, superparamagnetischen oder ferromagnetischen Material angeordnet ist, wobei zwischen dem mindestens einen Magneten (11) und dem mindestens einen Körper (12) eine Anziehungskraft besteht.

11. Einrichtung nach Anspruch 8, **dadurch gekennzeichnet, dass** am Deckel (3) zumindest ein Körper (11) aus einem magnetischen, paramagnetischen, superparamagnetischen oder ferromagnetischen Material angeordnet ist, und wobei an einer dem Deckel (3) gegenüberliegenden Wand (7a) des Gehäuses (7), zumindest ein Magnet (12) angeordnet ist, wobei zwischen dem mindestens einen Magneten (11) und dem mindestens einen Körper (12) eine Anziehungskraft besteht.

12. Einrichtung nach einem der vorhergehenden Ansprüche, **dadurch gekennzeichnet, dass** die mindestens eine Feder (4) eine Mehrzahl an Windungen (40) aufweist und so an einer Peripherie des Deckels (3) und des Bodens (2) angeordnet ist, dass das Reservoir (100) durch die Windungen (40) der mindestens einen Feder (4) gestützt wird.

13. Einrichtung nach einem der vorhergehenden Ansprüche, **dadurch gekennzeichnet, dass** die Einrichtung zumindest ein Spreizelement (13, 14) zum Stützen des Reservoirs (100) aufweist.

14. Einrichtung nach Anspruch 13, **dadurch gekennzeichnet, dass** das mindestens eine Spreizelement (13, 14) ein mit der Wand (1) des Reservoirs (100) verbundener Ring ist.

15. Einrichtung nach Anspruch 13, **dadurch gekennzeichnet, dass** das mindestens eine Spreizelement eine in einem Innenraum (103) des Reservoirs (100) angeordnete Platte (25) ist.

16. Einrichtung nach einem der vorhergehenden Ansprüche, **dadurch gekennzeichnet, dass** in einer dem Deckel (3) gegenüberliegenden Wand (7a) des Gehäuses (7) eine Öffnung (15) angebracht ist.

17. Einrichtung nach Anspruch 16, **dadurch gekennzeichnet, dass** die Einrichtung eine bewegliche Scheibe (16) aufweist, die in einem Innenraum (102) des Gehäuses (7) angeordnet ist und der Öffnung (15) gegenüberliegt, wobei die Scheibe (16) zur Abdichtung des Gehäuses über eine Membran (17) oder einen Balg (17) mit dem Gehäuse (7) verbunden ist, so dass sie zum Spannen der mindestens einen Feder (4) manuell gegen den Deckel (3) drückbar ist.

18. Einrichtung nach einem der vorhergehenden Ansprüche, **dadurch gekennzeichnet, dass** eine elastische Pumpkammer in oder an der der Wand des Gehäuses vorgesehen ist, die über ein Rückschlagventil mit der Atmosphäre verbunden ist und über ein weiteres Rückschlagventil mit einem Innenraum des Gehäuses derart verbunden ist, dass die Rückschlagventile Strömung von der Atmosphäre in die elastische Pumpkammer und aus der Pumpkammer in den Innenraum des Gehäuses erlauben und Strömung in Gegenrichtung sperren.

## Claims

1. A drainage device, with
- a tubular reservoir (100) comprising a flexible wall (1) and a rigid cover (3) and a rigid bottom (2), and
- at least one spring (4) which exerts a force pushing the cover (3) and the bottom (2) apart from each other, wherein the cover (3) is movable towards the bottom (2) to bring the spring into an initial position,
**characterized in**
**that** the device comprises one or more magnets (11, 12) configured to apply an additional force on the reservoir (100) in such a manner that a resulting force, pushing the cover (3) and the bottom (2) apart over that part of the spring deflection, does not exceed 50%, 40%, 30%, 20%, 10%, 5%, or 1% of the average amount of the force over that part of the spring deflection, or the force corresponds to a negative pressure in the reservoir that is in the range of between 20 and 40 mmHg (2.67 to 5.33 kPa) over that part of the spring deflection of the spring between the initial and final positions of the spring.

2. The device according to claim 1, **characterized in that** the at least one spring (4) is preloaded such that the deflection between the initial position and an end position of the at least one spring (4) predetermined by the length of the reservoir (100) constitutes only a part of the total spring deflection of the at least one spring (4).

3. The device according to claim 1 or 2, **characterized in that** the tubular reservoir (100) has at least one connector (5, 6) for connecting a hose.

4. The device according to claim 3, **characterized in that** the reservoir (100) is arranged in a housing (7) of the device, and that the at least one connector (5, 6) penetrates the housing (7) and is tightly connected to the housing (7) and the reservoir (100) or is sealed into them by means of sealing materials.

5. The device according to claim 4, **characterized in that** the housing (7) has at least one connector (8) via which ambient air can be let in and let out of an internal space (102) of the housing (7) between a wall (7a) of the housing (7) and a wall of the reservoir (100).

6. The device according to claim 5, **characterized in that** in the at least one connector (8) a flow resistor (9) is arranged which (9) limits the volumetric flow of the ambient air.

7. The device according to claim 6, **characterized in that** the flow resistor (9) is formed by one of the following elements:
- a nozzle,
- an element consisting of a plurality of nozzles,
- a porous element,
- a membrane having one or a plurality of through-holes,
- a membrane made of a gas-permeable material or comprising a gas-permeable material
- a membrane made of silicone rubber or comprising silicone rubber.

8. The device according to one of the preceding claims, **characterized in that** the bottom (2) of the reservoir (100) is firmly attached to a wall (7b) of the housing (7) and the cover (3) is movably arranged relative to the housing (7) in the housing (7).

9. The device according to claim 8, **characterized in that** at least one magnet (11) is arranged on the cover (3), and wherein at least one further magnet (12) is arranged on a wall (7a) of the housing (7) positioned opposite the cover (3), wherein the at least one magnet (11) and the at least one further magnet (12) are oriented such that they attract one another.

10. The device according to claim 8, **characterized in that** at least one magnet (11) is arranged on the cover (3), and wherein at least one body made of a magnetic, paramagnetic, superparamagnetic or ferromagnetic material is arranged on a wall (7a) of the housing (7) positioned opposite the cover (3), wherein an attractive force exists between the at least one magnet (11) and the at least one body (12).

11. The device according to claim 8, **characterized in that** at least one body (11) made of a magnetic, paramagnetic, superparamagnetic or ferromagnetic material is arranged on the cover (3), and wherein at least one magnet (12) is arranged on a wall (7a) of the housing (7) positioned opposite the cover (3), wherein an attractive force exists between the at least one magnet (12) and the at least one body (11).

12. The device according to any one of the preceding claims, **characterized in that** the at least one spring (4) has a plurality of coils and is arranged at a periphery of the cover (3) and the bottom (2) such that the reservoir (100) is supported by the coils (40) of the at least one spring (4).

13. The device according to any one of the preceding claims, **characterized in that** the device comprises at least one spreader element (13, 14) for supporting the reservoir (100).

14. The device according to claim 13, **characterized in that** the at least one spreader element (13, 14) is a ring connected to the wall (1) of the reservoir (100).

15. The device according to claim 13, **characterized in that** the at least one spreader element is a plate (25) arranged in an inner space (103) of the reservoir (100).

16. The device according to any one of the preceding claims, **characterized in that** an opening (15) is provided in a wall (7a) of the housing (7) opposite the cover (3).

17. The device according to claim 16, **characterized in that** the device comprises a movable washer (16) arranged in an internal space (102) of the housing (7) and opposite the opening (15), wherein the washer (16) is connected to the housing (7) via a membrane (17) or a bellows (17) for sealing the housing so that it can be pressed manually against the cover (3) for tensioning the at least one spring (4).

18. The device according to one of the preceding claims, **characterized in that** in or on the wall of the housing the device comprises an elastic pump chamber, which is connected to the atmosphere via a non-return valve and is connected to an internal space of the housing via a further non-return valve in such a manner that the non-return valves allow flow from the atmosphere into the elastic pump chamber and from the pump chamber into the internal space of the housing and block flow in the opposite direction.

## Revendications

1. Dispositif pour le drainage avec:
- un réservoir tubulaire (100) comprenant une paroi flexible (1) et un couvercle rigide (3) et une base rigide (2), et
- au moins un ressort (4) qui exerce une force poussant le couvercle (3) et la base (2) à l'écart, le couvercle (3) étant mobile dans la direction du fond (2) pour amener le ressort dans une position initiale,
est **caractérisé en ce**
**que** le dispositif comprend un ou plusieurs aimants (11, 12) configurés pour exercer une force supplémentaire sur le réservoir (100) de sorte qu'une force résultante poussant le couvercle (3) et la base (2) à l'écart sur cette partie de l'allongement du ressort ne s'écarte pas de plus de 50%, 40%, 30%, 20%, 10%, 5% ou 1% de la force moyenne sur cette partie de l'allongement du ressort, ou la force correspond à une pression négative dans le réservoir qui est dans la gamme de 20 à 40 mmHg (2,67 à 5,33 kPa) sur cette partie de l'allongement du ressort entre les positions initiale et finale du ressort.

2. Dispositif selon la revendication 1 est **caractérisé en ce que** le au moins un ressort (4) est précontraint de telle sorte que la course entre la position initiale et une position finale du au moins un ressort (4) prédéterminée par la longueur du réservoir (100) constitue seulement une partie de l'allongement total du au moins un ressort (4).

3. Dispositif selon la revendication 1 ou 2 est **caractérisé en ce que** le réservoir tubulaire (100) comporte au moins un raccord (5, 6) pour le raccordement d'un tuyau.

4. Dispositif selon la revendication 3 est **caractérisé en ce que** le réservoir (100) est disposé dans un boîtier (7) du dispositif, et **en ce que** l'au moins un raccord (5, 6) traverse le boîtier (7) et est relié de manière étanche au boîtier (7) et au réservoir (100) ou est scellé dans ceux-ci par des moyens d'étanchéité.

5. Dispositif selon la revendication 4 est **caractérisé en ce que** le boîtier (7) présente au moins un raccord (8) par lequel l'air ambiant peut être admis dans et évacué d'un espace intérieur (102) du boîtier (7) entre une paroi (7a) du boîtier (7) et une paroi (3) du réservoir (100).

6. Dispositif selon la revendication 5 est **caractérisé en ce qu'**une résistance à l'écoulement (9) est disposée dans l'au moins un raccord (8) qui limite le débit volumique de l'air ambiant.

7. Dispositif selon la revendication 6 est **caractérisé en ce que** la résistance à l'écoulement (9) est formée par l'un des éléments suivants:
- une buse,
- un élément constitué d'une pluralité de buses,
- un élément poreux,
- une membrane comportant un ou plusieurs trous de passage,
- une membrane constituée de ou comprenant un matériau perméable aux gaz,
- une membrane constituée de ou comprenant du caoutchouc de silicone.

8. Dispositif selon l'une des revendications précédentes est **caractérisé en ce que** la base (2) du réservoir (100) est reliée de manière fixe à une paroi (7b) du boîtier (7) et le couvercle (3) est disposé de manière mobile dans le boîtier (7) par rapport au boîtier (7).

9. Dispositif selon la revendication 8 est **caractérisé en ce qu'**au moins un aimant (11) est disposé sur le couvercle (3), et dans lequel au moins un autre aimant (12) est disposé sur une paroi (7a) du boîtier (7) opposée au couvercle (3), dans lequel le au moins un aimant (11) et le au moins un autre aimant (12) sont orientés de telle sorte qu'ils s'attirent mutuellement.

10. Dispositif selon la revendication 8 est **caractérisé en ce qu'**au moins un aimant (11) est disposé sur le couvercle (3), et dans lequel au moins un corps fait d'un matériau magnétique, paramagnétique, superparamagnétique ou ferromagnétique est disposé sur une paroi (7a) du boîtier (7) opposée au couvercle (3), dans lequel une force d'attraction existe entre le au moins un aimant (11) et le au moins un corps (12).

11. Dispositif selon la revendication 8 est **caractérisé en ce qu'**au moins un corps (11) en un matériau magnétique, paramagnétique, superparamagnétique ou ferromagnétique est disposé sur le couvercle (3), et dans lequel au moins un aimant (12) est disposé sur une paroi (7a) du boîtier (7) opposée au couvercle (3), une force d'attraction existant entre le au moins un aimant (11) et le au moins un corps (12).

12. Dispositif selon l'une quelconque des revendications précédentes est **caractérisé en ce que** l'au moins un ressort (4) comprend une pluralité de spires (40) et est disposé à une périphérie du couvercle (3) et de la base (2) de sorte que le réservoir (100) soit supporté par les spires (40) de l'au moins un ressort (4).

13. Dispositif selon l'une quelconque des revendications précédentes est **caractérisé en ce que** le dispositif comprend au moins un élément écarteur (13, 14) pour supporter le réservoir (100).

14. Dispositif selon la revendication 13 est **caractérisé en ce que** le au moins un élément écarteur (13, 14) est un anneau relié à la paroi (1) du réservoir (100).

15. Dispositif de la revendication 13 est **caractérisé en ce que** le au moins un élément écarteur est une plaque (25) disposée dans un espace intérieur (103) du réservoir (100).

16. Dispositif selon l'une des revendications précédentes est **caractérisé en ce qu'**une ouverture (15) est prévue dans une paroi (7a) du boîtier (7) opposée au couvercle (3).

17. Dispositif selon la revendication 16 est **caractérisé en ce que** le dispositif comprend un disque mobile (16) disposé dans un espace intérieur (102) du boîtier (7) et positionné en face de l'ouverture (15), le disque (16) étant relié au boîtier (7) par l'intermédiaire d'une membrane (17) ou d'un soufflet (17) pour l'étanchement du boîtier de sorte qu'il puisse être pressé manuellement contre le couvercle (3) pour tendre le au moins un ressort (4).

18. Dispositif selon l'une quelconque des revendications précédentes est **caractérisé en ce qu'**une chambre de pompage élastique est prévue dans ou sur la paroi du boîtier, reliée à l'atmosphère par un clapet anti-retour et reliée à l'espace intérieur du boîtier par un autre clapet anti-retour, de sorte que les clapets anti-retour permettent un écoulement de l'atmosphère vers la chambre de pompage élastique et de la chambre de pompage vers l'espace intérieur du boîtier et bloquent l'écoulement dans la direction opposée.
